# DEMANDE DE BREVET EUROPEEN

(11) **EP 1 452 156 A1**
(43) Date de publication de la demande: **01.09.2004**
(21) Numéro de dépôt: 04370005.3
(22) Date de dépôt: 25.02.2004
(51) Int. Cl.: A61F 13/02

(54) **Membrane multicouche flexible adhésive à base de non-tissé et de silicones médicaux**

(30) Priorité: 27.02.2003 FR 0302410
(71) Demandeur: Dufour, Catherine, 59100 Roubaix (FR)
(72) Inventeur: Dufour, Catherine, 59100 Roubaix (FR)
(74) Mandataire: Hennion, Jean-Claude

(57) **Abrégé**

Membrane pour application sur la peau humaine comportant deux couches, une première couche (4) flexible, présentant une face intérieure (4a) et une face extérieure (4b), et une deuxième couche adhésive (5), caractérisée en ce que la première couche (4) comprend un textile non-tissé et est enduite, sur sa face intérieure (4a), de la deuxième couche adhésive (5) et sur sa face extérieure (4b), d'une couche à base d'élastomère (6).

## Description

L'invention se rapporte, d'une manière générale, à une membrane synthétique souple adhésive visant à masquer temporairement des imperfections cutanées, notamment du visage, ou à reproduire l'apparence physique de certains éléments manquants du visage, ou encore à constituer le complément d'une prothèse, notamment faciale (épithèse).

Il existe un besoin croissant, ressenti par les personnes présentant des imperfections cutanées, d'améliorer leur apparence physique, besoin qui n'est pas complètement satisfait actuellement. Dans notre société, l'apparence physique présente une importance indéniable. De ce fait, une personne présentant un défaut visible, congénital, de développement ou acquis, peut rencontrer des difficultés dans son intégration sociale. De tels défauts peuvent affecter également l'image de soi de la personne concernée. Comme l'apparence et l'expression du visage sont très visibles et constituent des moyens essentiels de communication, les imperfections situées dans ces régions sont encore plus traumatisantes que celles concernant d'autres parties du corps.

Dans le domaine des altérations physiques, actuellement, seules les altérations lourdes et très visibles dues aux ablations chirurgicales des cancers ORL sont traitées par le port d'épithèses. Les problèmes moins lourds, ou ne concernant que la surface cutanée, sans organe manquant, ne trouvent que des solutions partielles et imparfaites par : les pansements, le maquillage, les tatouages ou la chirurgie esthétique.

Ainsi, les pansements, qui sont des dispositifs adhérents permettant de protéger une plaie de l'infection et de favoriser sa cicatrisation, ont comme principal atout d'être bon marché. Lorsque utilisés pour camoufler les imperfections cutanées, les pansements ne permettent pas véritablement d'améliorer l'apparence physique, car, si on ne voit plus directement le défaut, on remarque le pansement. De plus, les pansements ne peuvent intéresser que les personnes dont les cicatrices sont récentes et vont disparaître en quelques jours ou quelques semaines.

Le document US-5891076 décrit un pansement pour cicatrices hypertrophiques comprenant un support flexible recouvert d'une enduction double face avec un gel silicone adhésif, gel qui forme deux couches continues, de part et d'autre dudit support. Le support, constitué de fibres textiles tissées, non-tissées ou de film plastique perforé, est perméable aux liquides, ce qui permet au gel silicone de pénétrer ledit support, au moins ponctuellement, de façon à assurer une liaison transversale des deux couches de gel à travers le support. La face extérieure du pansement comprend un film de polyuréthane.

Le document WO-A-02/45698 décrit un pansement utilisé comme masque facial dans le traitement des cicatrices, comprenant un support textile présentant, sur la face intérieure, une enduction avec un gel silicone. Le support textile est constitué de fibres tissées élastiques, par exemple fibres de polyamide.

Le document US-5919476 décrit un pansement pour le traitement des cicatrices, comprenant trois couches : une première couche en silicone adhésif, qui adhère à la peau ; une deuxième couche en contact avec la première et lui servant de support, comprenant un matériau textile à mailles (polyester) ; une troisième couche solidaire avec les deux autres, recouvrant et obstruant les mailles de la deuxième couche, ladite troisième couche étant constituée, de préférence, en silicone non-adhésif.

Malgré les qualités performantes de ces pansements en ce qui concerne le traitement des cicatrices, leur apparence externe n'est pas soignée, ce qui les rend inappropriés à un usage cosmétique, pour lequel ils ne sont pas conçus.

En ce qui concerne le maquillage, il n'existe que quelques marques spécialisées dans la correction des imperfections cutanées : la gamme COUVRANCE d'Avène et les marques COVERDERM® et COVERMARK™. Elles proposent des gammes de fonds de teint, sticks correcteurs et poudres très couvrantes pour le visage et le corps. Ces produits nécessitent du temps pour se maquiller et un bon doigté pour appliquer les fards de façon efficace. De plus, ils ne masquent pas des défauts plus prononcés, n'ont pas une longue tenue, présentent l'inconvénient de tâcher et doivent être nettoyés quotidiennement. Enfin, ces produits ne sont pas adaptés aux personnes qui transpirent beaucoup ou qui sont impliquées dans des activités physiques soutenues.

Certains produits proposés cherchent à combiner les avantages des pansements et du maquillage. Ainsi, le document US-5586971 décrit un produit destiné à couvrir des blessures ou des imperfections cutanées, et comportant une bande adhésive présentant une face supérieure et une périphérie. La bande comprend une couche de maquillage adapté à la couleur de peau souhaitée, appliquée sur la face supérieure, ainsi que sur la surface de la peau adjacente à ladite bande. La bande comprend une couche supérieure en papier ayant rôle de support, une couche adhésive sous-jacente et une troisième couche qui s'applique par-dessus la partie de peau à couvrir de façon non-adhérente. Ladite troisième couche est de surface plus réduite que la couche adhésive, créant ainsi un anneau périphérique adhésif, permettant de coller la bande à la peau, autour de la blessure ou du défaut à masquer.

Le tatouage professionnel permanent, représentant l'action de marquer ou orner une partie du corps de dessins ou d'inscriptions par l'introduction de substances colorantes sous la peau, au moyen d'une aiguille, est également utilisé dans le but d'améliorer l'apparence physique d'une personne, soit en couvrant ou masquant des cicatrices et d'autres imperfections cutanées, soit en simulant des parties manquantes du visage, comme les sourcils. Cependant, cette option n'est pas totalement efficace et présente de nombreuses conséquences indésirables : elle est douloureuse, peut conduire à contracter des maladies transmissibles comme le SIDA ou l'hépatite, et un tatouage permanent ne peut pas être modifié pour suivre les changements de tons de la peau dus au vieillissement.

Dans cette perspective, il apparaît que le tatouage temporaire, impliquant le transfert sur la peau d'une image colorée à partir d'un support pré imprimé, peut se présenter comme une solution plus intéressante, comme prouvé par la grande diversité de produits de ce type disponibles récemment sur le marché.

Ainsi, le document US-A-2001/0023328 décrit une méthode de fabrication d'un tatouage temporaire. En bref, la partie du corps qui sera ultérieurement enlevée suite à un acte chirurgical, ou bien des éléments équivalents situés du côté opposé à celui qui doit être reproduit, sont photographiés et une applique temporaire ou tatouage est fabriqué à partir de l'image digitale obtenue après avoir scanné la photo. Le tatouage temporaire obtenu comprend un support, une image et un adhésif.

Le document US-A-2002/0110672 décrit un tatouage temporaire opaque destiné à masquer des imperfections cutanées pendant trois à sept jours, comportant un substrat composé d'un film polymérique et portant, sur une de ses faces, une image de la peau humaine imprimée, et sur l'autre face, un adhésif sensible à la pression et perméable à l'humidité. Une couche de film jetable est placée au-dessus de la couche adhésive. Un support, fabriqué en film polymérique ou papier, est placé contre l'image.

Cependant, il apparaît que le tatouage temporaire n'est pas totalement efficace pour couvrir certains défauts, et, de plus, il a une durée de vie limitée et ne peut être utilisé qu'une seule fois.

La chirurgie esthétique utilisant ou non un laser permet de changer l'apparence physique et donc de faire disparaître certains défauts, notamment du visage, et ceci, éventuellement, en dehors de toute nécessité thérapeutique. Une opération de chirurgie esthétique demeure toujours un acte chirurgical délicat dont la réussite dépend pour l'essentiel de l'habileté du chirurgien. A ceci, il faut rajouter le coût non-négligeable, les risques liés à l'anesthésie, le fait qu'il n'y a pas de garantie de résultat et l'inconfort physique post-opératoire.

Les épithèses sont des prothèses faciales en silicone obtenues par moulage, permettant de reconstituer un organe manquant (oreille, nez, oeil, ...) et de redonner au patient l'harmonie initiale de son visage. Les épithèses ne sont pas adaptées aux défauts moins importants et aux problèmes non pris en charge par le secteur médical.

Le but de la présente invention est de proposer une alternative efficace, facile à utiliser et non-définitive, aux techniques médicales actuelles, ainsi qu'une solution plus performante par rapport aux utilisations de produits temporaires, destinées à améliorer l'apparence physique des personnes présentant des imperfections cutanées.

Ce but est atteint au moyen d'une membrane pour application sur la peau humaine, comportant deux couches, à savoir une première couche flexible présentant une face intérieure et une face extérieure, et une deuxième couche adhésive, la première couche comprenant un textile non-tissé et est enduite, sur sa face intérieure, de la deuxième couche adhésive et sur sa face extérieure, d'une couche à base d'élastomère, ladite membrane comprenant des moyens d'opacification et/ou des moyens intrinsèques et/ou extrinsèques de coloration de la couche à base d'élastomère, de sorte que cette membrane présente des caractéristiques semblables à celles de la peau humaine, notamment en ce qui concerne la couleur, l'aspect, l'élasticité.

Selon diverses réalisations, cette membrane présente les caractères suivants, le cas échéant combinés :
- les moyens d'opacification consistent en l'incorporation, dans l'élastomère silicone constituant la couche à base d'élastomère, de charges comprenant du talc ;
- les moyens intrinsèques comprennent des pigments incorporés dans l'élastomère silicone constituant la couche à base d'élastomère, avant réticulation ;
- les moyens extrinsèques comprennent des pigments rapportés sur la couche à base d'élastomère ;
- les moyens extrinsèques de coloration de la couche à base d'élastomère comprennent une impression par transfert thermique sur ladite couche d'une photo d'une partie du corps humain, notamment du visage, comprenant des éléments équivalents à ceux destinés à être reproduits sur ladite couche ;
- le textile non-tissé comprend un polyester ou un polypropylène ;
- la couche à base d'élastomère comprend un élastomère silicone ;
- la deuxième couche adhésive comprend un gel silicone ;
- l'épaisseur de la membrane est sensiblement comprise entre 0,25 mm et 2 mm ;
- elle comprend des moyens permettant une certaine perméabilité à l'eau, à la sueur et à la vapeur à ladite membrane.

L'invention se rapporte, selon un deuxième aspect, à l'utilisation d'une membrane telle que présentée ci-dessus pour la fabrication de patchs esthétiques.

L'invention se rapporte, selon un troisième aspect, à un procédé de fabrication d'une membrane telle que présentée ci-dessus, ce procédé comprenant une étape d'enduction double face de la première couche en non-tissé avec au moins une couche adhésive et avec au moins une couche à base d'élastomère, suivie des étapes suivantes : traitement thermique ; calandrage ; micro perforations.

L'invention fournit ainsi un produit destiné à être collé sur la peau dans le but de masquer temporairement certaines imperfections cutanées, par exemple : cicatrices, brûlures, angiomes, naevus, dépigmentations, couperose, rougeurs post-opératoires, boutons, tout en les protégeant du soleil si besoin.

L'invention fournit également un produit destiné à reproduire l'apparence de certains éléments manquants du visage.

Dans une réalisation, l'invention fournit un produit qui se présente comme un patch esthétique, de couleur et aspect peau, fin, souple et auto-adhérent. Par le terme « patch esthétique » on entend, dans le cadre de la présente invention, une pièce prédécoupée dans la membrane présentée ci-dessus de taille et de forme définies, que l'on applique sur la peau à l'aide de la couche adhésive incorporée, afin de protéger ou de dissimuler une imperfection cutanée ou de reproduire l'apparence de certains éléments manquants du visage.

Dans une autre réalisation, l'invention fournit un support de fixation pour epithèses présentant une adhérence améliorée par rapport aux supports connus.

L'invention sera mieux comprise à la lecture de la description suivante faite en référence aux dessins annexés, dans lesquels :
- la figure 1 est une section transversale de la membrane comprenant un non-tissé et une couche adhésive ;
- la figure 2 est une section transversale d'un patch esthétique;
- la figure 3 est une section transversale d'un support de fixation d'une épithèse.

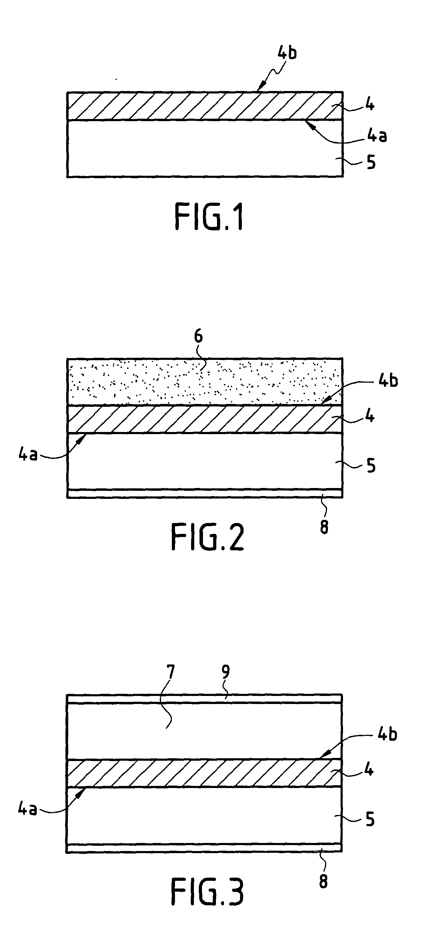

Comme montré dans la figure 1, la membrane 1 comprend deux couches, une première couche 4 en textile non-tissé, présentant une face intérieure 4a et une face extérieure 4b, et recouverte sur la face intérieure 4a par enduction avec une couche adhésive 5. La couche adhésive 5 comprend, de préférence, un gel silicone adhésif.

Dans une variante de réalisation, la première couche 4 de la membrane 2 est recouverte, sur sa face extérieure 4b, par enduction, avec une couche à base d'élastomère 6 (fig. 2), les trois couches 6, 4 et 5 formant la membrane 2, utilisée, avantageusement, pour fabriquer des patchs esthétiques. Dans un mode de réalisation, la couche 6 comprend un élastomère silicone. Un film anti-adhésif pelable et jetable 8 peut être apposé contre la couche adhésive 5.

Dans une autre variante de réalisation, la première couche 4 de la membrane 3 est recouverte, sur sa face extérieure 4b, par enduction, avec une couche adhésive 7 (fig. 3), les trois couches 7, 4 et 5 formant la membrane 3, utilisée, avantageusement, comme support de fixation des épithèses. La couche adhésive 7 comprend, dans une réalisation, le même gel silicone adhésif que celui utilisé pour la couche 5. Un film anti-adhésif pelable et jetable (8, 9) est apposé par-dessus chacune des deux couches adhésives 5 et 7, respectivement.

La membrane multicouches flexible peut être fabriquée en plusieurs étapes, décrites ci-dessous :
a - déroulage de la première couche 4 de support;
b - contrôle de la tension de cette première couche;
c - alimentations en élastomère 6 et en adhésif 5, 7 ;
d - enduction à la racle sur les deux faces 4a et 4b de la première couche 4, avec contrôle précis de l'épaisseur enduite ;
e - traitement thermique sur rame chauffante, comprenant un passage de dix min minimum à cent degrés celsius ;
f - calandrage « effet peau » ;
g - micro-perforations ;
h - contre-collage d'un film anti-adhésif pelable et jetable 8 ou 9 par-dessus les couches adhésives 5 et 7, respectivement ;
i - enroulage ;
j - découpage ;
k - emballage ;
l - stérilisation.

Un textile non-tissé est une étoffe comprenant des fibres ou filaments disposés de manière à créer une surface et dont la cohésion est obtenue par des moyens autres que ceux des textiles traditionnels. La première couche en textile non-tissé 4 assure la cohésion de l'ensemble de couches constituant la membrane et apporte un renforcement mécanique à celle-ci. Surtout, le non-tissé sert de support flexible à l'enduction double face. Il doit présenter les caractéristiques suivantes :
- souplesse,
- finesse et déformabilité pour permettre de restituer l'effet d'élasticité de la couche à base d'élastomère 6 assurant la face peau de la membrane,
- très bonne résistance au déchirement et au vieillissement,
- caractère isotrope,
- inertie chimique.

Dans une variante de réalisation, la première couche 4 en non-tissé est élaborée au moins en partie à base de polyester. Le polyester présente l'avantage de résister aux températures élevées, nécessaires pour réaliser la réticulation de la couche à base d'élastomère 6 ; il peut être ainsi adapté à une production industrielle. Cependant, le polyester possède une souplesse limitée.

Dans une autre variante de réalisation, la première couche 4 en non-tissé est élaborée au moins en partie à base de polypropylène. Le polypropylène est généralement plus souple que le polyester, mais ne résiste pas aux hautes températures.

La couche à base d'élastomère 6 comprend, de préférence, un élastomère silicone médical. Il doit répondre à plusieurs critères: souplesse, élasticité, propriétés de surface, résistance élevée à l'eau et à la sueur, résistance au vieillissement, à l'ozone et aux rayons UV. Sont utilisés avantageusement des silicones RTV-2 (vulcanisation à température ambiante lors du mélange de deux composants) réticulant par addition. Les avantages de la réticulation par addition sont :
- la possibilité d'accélérer la réaction en augmentant la température ;
- l'absence du retrait qui ferait s'enrouler la membrane sur elle-même ;
- l'absence d'obligation du contrôle contraignant de l'humidité pendant la réticulation.

Ce mode de réticulation est basé sur l'addition des groupes de hydrure de silyle (-SiH) aux groupes vinyle (CH₂=CH-) attachés au silicone par l'intermédiaire d'un catalyseur à base de platine. La réaction a lieu à température ambiante quand on utilise des complexes d'oléfines ou de métaux contenant du platine.

Un autre composant de la couche d'élastomère 6 est représenté par les charges incorporées.

Dans une variante de réalisation, les charges incorporées aux élastomères silicones comprennent du talc. Le talc, occupant une position médiane entre les charges renforçantes et non renforçantes, permet d'augmenter la viscosité du silicone : celui-ci ne traverse pas la première couche 4 en non-tissé et l'épaisseur enduite est mieux contrôlée. Ceci évite un endurcissement prématuré et donc augmente la durée de vie du produit. De plus, le talc permet de donner un aspect opaque au produit final (couche à base d'élastomère 6), afin qu'on ne distingue pas les défauts de la peau à travers la membrane, tout en gardant une certaine transparence de surface. Aussi, son addition confère à la membrane une meilleure résistance, tout en préservant une élasticité optimale pour que le film puisse suivre les mouvements de la peau.

La couche à base d'élastomère 6 doit avantageusement être de couleur semblable à celle de la peau humaine. Dans une variante de réalisation, ceci est obtenu par des moyens intrinsèques. La coloration intrinsèque de la couche à base d'élastomère 6 est obtenue par incorporation de pigments appropriés au mélange base/catalyseur/talc, avant réticulation. Un dosage précis de pigments spéciaux « effet peau » est nécessaire afin d'obtenir une teinte la plus proche possible de celle de la couleur de peau souhaitée.

Dans une autre variante de réalisation, la couche à base d'élastomère 6 présente une couleur et un aspect semblables à ceux de la peau humaine grâce à des moyens extrinsèques, comprenant l'utilisation de pigments extrinsèques et/ou l'impression par transfert thermique sur un élastomère d'une photo d'une partie du corps humain, notamment du visage, comprenant des éléments équivalents à ceux destinés à être reproduits sur ledit silicone. En pratique, la peau est photographiée du côté opposé au défaut pour obtenir la couleur et le grain de peau de la personne concernée. L'image obtenue est traitée sur ordinateur afin de réaliser, notamment, les retouches nécessaires, l'extrapolation pour grandes surfaces, l'adaptation à la forme du patch. Cette image est ensuite appliquée à l'élastomère par impression par transfert thermique.

La membrane multicouches flexible possède une bonne adhérence à la peau. Ceci est obtenu par enduction de la face intérieure 4a de la première couche 4 support en textile non-tissé avec un adhésif 5, de préférence un gel silicone médical.

Du fait de son intime adhérence à la peau, la couche intérieure de la membrane, à base de gel silicone, doit être le moins agressive possible envers la peau. Ceci est particulièrement important quand la membrane est destinée à couvrir des zones brûlées ou irritées, surtout pour des périodes de temps plus longues (plusieurs jours, voir semaines). L'innocuité des silicones adhésifs est en elle-même connue. Ainsi, l'immunogénicité des silicones adhésifs est négligeable, comme le montrent de nombreuses études, par exemple : *Burns* 2001, 27(3) :205-14 ; *Cutis* 1995, 56(1) :65-7. Les silicones adhésifs possèdent non seulement une très faible immunogénicité, mais en outre il semble qu'ils encouragent la guérison des cicatrices hypertrophiques ou chéloïdes, le mécanisme impliqué n'étant pas encore élucidé.

L'épaisseur de la membrane dépend directement du site d'application de cette membrane. En général, l'épaisseur est comprise entre 0.25 et 2 mm environ.

La membrane multicouches flexible décrite peut être utilisée pour la fabrication des patchs esthétiques, destinés à masquer ou protéger des imperfections cutanées; elle peut également être utilisée pour la fabrication des supports de fixation des épithèses, présentant une adhérence améliorée par rapport aux produits connus.

La membrane multicouches flexible va être maintenant illustrée plus en détail par les exemples suivants.

### Exemple 1. Fabrication d'une membrane multicouches flexible adhésive

### A. Première couche en non-tissé 4

Dans une variante de réalisation, le non-tissé utilisé est représenté par du polyester, comme celui fabriqué sous la référence N5N320 par POLYMER GROUP, présentant les caractéristiques suivantes : épaisseur : 0,13 mm ; résistance à la rupture MD : 42 N/5cm ; résistance à la rupture CD : 35 N/5cm ; allongement à la rupture MD : 14% ; allongement à la rupture CD : 20% ; perméabilité à l'air (8700 l/m²/s) et à l'eau (1250 l/m²/s).

Dans une autre variante de réalisation, le non-tissé utilisé est représenté par du polypropylène issu de la société DOUNOR et portant les références PPSS14EOS, présentant les caractéristiques suivantes : épaisseur : 0,1 mm ; résistance à la rupture MD : 28 N ; résistance à la rupture CD : 18N ; allongement à la rupture MD : 75% ; allongement à la rupture CD : 85%.

### B. Préparation de la couche à base d'élastomère silicone 6

Les silicones médicaux RTV-2 sont composés d'une base et d'un catalyseur à mélanger dans les conditions préconisées par le fabricant. Le mélange obtenu sera polymérisé soit à température ambiante pendant 4 h, soit à 100°C pendant environ 15 min. Un accélérateur est ajouté afin d'augmenter la vitesse de réticulation et de rendre ainsi possible une fabrication industrielle.

Pour la préparation de la couche à base d'élastomère silicone 6, peuvent être utilisés les produits suivants: Silastic® MDX4-4210 Medical Grade Elastomer, fabriqué par DOW CORNING; Dropstil 557, fabriqué par PREVENT TRANSFORMATION ; Prosthetic Silicone External A-220, commercialisé par TECHNOVENT.

En tant qu'adjuvants utilisés pour la préparation de la couche à base d'élastomère silicone, le talc (silicate de calcium) peut être obtenu en pharmacie ou fourni par TECHNOVENT, et les pigments intrinsèques spéciaux « effet peau », fabriqués, par exemple, par FACTOR II (Functional Intrinsic Skin Colors).

### C. Préparation de la couche à base de gel silicone adhésif 5, 7

Les silicones adhésifs se préparent d'une manière similaire à celle décrite plus haut, pour les élastomères silicones en général. Dans le cas particulier des silicones adhésifs, la réticulation peut être arrêtée au stade de gel, il en résulte des produits de vulcanisation collants, faiblement réticulés, qui se caractérisent par une bonne adhésion inhérente. Le gel silicone (PREVENT TRANSFORMATION) se présente comme un gel translucide, constitué de deux parties à mélanger dans les proportions 50/50. Après avoir été déposé par enduction sur la face intérieure 4a de la première couche 4 en non-tissé, la réticulation est obtenue par un passage de 15 min à 100°C. La pellicule ainsi formée reste adhésive et pourra être déposée sur un film plastique siliconé pour le transport et le stockage. Les propriétés adhésives de ce gel sont particulièrement élevées et elles pourront être restituées, si le besoin se fait sentir après plusieurs utilisations, par un nettoyage avec un agent détergent doux qui éliminera l'encrassement.

### D. Calandrage

Le calandrage est une technique de laminage d'une matière thermoplastique entre plusieurs cylindres parallèles ; il peut se faire à froid ou à chaud. Dans une variante de réalisation, la membrane multicouches 2 est laminée entre deux cylindres, le rouleau supérieur étant gravé de façon à donner, par impression, un aspect peau à la couche à base d' élastomère 6. Dans une variante préférée de réalisation, le calandrage s'effectue à chaud, à la fin du traitement thermique de réticulation, après apposition d'un film pelable 8 sur la couche adhésive 5.

### E. Micro-perforations

Les micro-perforations de la membrane sont réalisées à l'aide d'aiguilles fines, les trous obtenus dans la couche à base d'élastomère 6 ayant un diamètre de quelques microns. Ces micro-perforations confèrent à la membrane une certaine perméabilité à l'air, à la sueur et à la vapeur d'eau. Pour permettre à la peau de respirer normalement, la membrane doit avoir une perméabilité à l'air d'environ 6 x 10⁻⁷ L/cm²/min. Cette porosité est obtenue par une micro-perforation d'un certain nombre de trous par cm², nombre qui est à définir après essais pour chaque type de support.

### Exemple 2. Utilisation de la membrane multicouches flexible adhésive pour la fabrication de patchs esthétiques

Après fabrication de la membrane, des finitions manuelles sont possibles, à l'aide de pigments extrinsèques (FACTOR II) et du flocage (Flocking Rayon Fiber - TECHNOVENT), afin de maquiller la surface de la membrane et lui donner un aspect personnalisé : veines apparentes, poils, duvet, grains de beauté, pores, faux relief. Tous ces ajouts sont fixés à l'aide d'un adhésif (DOW CORNING Silastic® Medical Adhesive Type A) dilué dans un solvant (de type xylène) et matifiés grâce à une application de poudre.

Une fois les finitions réalisées, la membrane peut être découpée en morceaux de taille et forme différentes, qui vont constituer les patchs esthétiques proposés à la commercialisation.

Le patch esthétique décrit est utilisé en le collant sur la peau, à l'endroit d'une cicatrice, brûlure, angiome, ou d'une marque quelconque à dissimuler. Le patch peut rendre les mêmes services qu'un pansement : protéger la peau de salissures extérieures et des agressions de toute sorte, en évitant les infections, tout en présentant un aspect esthétique marqué. Il peut protéger un endroit précis de la peau des effets indésirables provoqués par l'exposition aux rayons solaires.

### Exemple 3. Utilisation de la membrane multicouches flexible adhésive comme complément des prothèses

La membrane synthétique adhésive peut être utilisée comme support d'une prothèse, notamment faciale (épithèse). Les prothèses existantes, réalisées en silicone moulé, sont fixées sur le visage par collage, vissage, à l'aide d'implants sous-cutanés avec fixation magnétique. Souvent, des difficultés sont rencontrées lors du collage des prothèses, notamment pour éliminer les restes de colle sur la peau et sur la prothèse pouvant conduire à l'encrassement de celle-ci. La membrane adhésive 2, comprenant une première couche support non-tissé 4 présentant une enduction double-face, avec un gel adhésif 5 sur sa face intérieure 4a et avec une couche à base d'élastomère 6 sur sa face extérieure 4b, se propose de remédier à ces inconvénients. Grâce à la couche à base d'élastomère 6, la membrane 2 support de prothèse peut être mieux adaptée au moulage, contribuant à un effet esthétique marqué. La couche adhésive 5 assure une fixation plus efficace des épithèses et conduit à l'obtention de bords beaucoup plus fins.

La membrane adhésive 2 peut aussi être utilisée comme surface d'une prothèse destinée à remplacer une partie manquante du corps, autre que du visage. En donnant une image fidèle de la peau, la couche à base d'élastomère 6 contribue à améliorer la qualité générale de la prothèse.

La membrane synthétique flexible adhésive décrite présente de nombreux avantages par rapport aux produits connus :
- adhérence améliorée à la peau ;
- facilité d'application à l'endroit voulu ;
- facilité d'entretien ;
- aucune gêne au porter et au retrait ;
- utilisations répétées et longue durée de vie ;
- apparence plus naturelle, grâce à un choix large de couleurs, adaptées aux carnations les plus courantes ;
- indolore et sans risques pour l'utilisateur.

La membrane décrite peut être enlevée de la partie du corps où elle a été appliquée aussi souvent que souhaité. La surface adhésive de la membrane est en contact avec un matériau anti-adhésif facilement pelable et qui y être ré appliqué après usage.

## Revendications

1. Membrane pour application sur la peau humaine comportant deux couches, une première couche (4) flexible, présentant une face intérieure (4a) et une face extérieure (4b), et une deuxième couche adhésive (5), **caractérisée en ce que** la première couche (4) comprend un textile non-tissé et est enduite, sur sa face intérieure (4a), de la deuxième couche adhésive (5) et sur sa face extérieure (4b), d'une couche à base d'élastomère (6), et **en ce que** ladite membrane comprend des moyens d'opacification et/ou des moyens intrinsèques et/ou extrinsèques de coloration de la couche à base d'élastomère (6), de sorte que ladite membrane présente des caractéristiques semblables à celles de la peau humaine, notamment en ce qui concerne la couleur, l'aspect, l'élasticité.

2. Membrane selon la revendication 1, **caractérisée en ce que** lesdits moyens d'opacification consistent en l'incorporation, dans l'élastomère silicone constituant la couche à base d'élastomère (6), de charges comprenant du talc.

3. Membrane selon l'une quelconque des revendications 1 ou 2, **caractérisée en ce que** lesdits moyens intrinsèques comprennent des pigments incorporés dans l'élastomère silicone constituant la couche à base d'élastomère (6), avant réticulation.

4. Membrane selon l'une quelconque des revendications 1 à 3, **caractérisée en ce que** lesdits moyens extrinsèques comprennent des pigments rapportés sur la couche à base d'élastomère (6).

5. Membrane selon l'une quelconque des revendications 1 à 4, **caractérisée en ce que** lesdits moyens extrinsèques de coloration de la couche à base d'élastomère (6) comprennent une impression par transfert thermique sur ladite couche d'une photo d'une partie du corps humain, notamment du visage, comprenant des éléments équivalents à ceux destinés à être reproduits sur ladite couche.

6. Membrane selon l'une quelconque des revendications 1 à 5, **caractérisée en ce que** le textile non-tissé comprend un polyester.

7. Membrane selon l'une quelconque des revendications 1 à 6, **caractérisée en ce que** le textile non-tissé comprend du polypropylène.

8. Membrane selon l'une quelconque des revendications 1 à 7, **caractérisée en ce que** la couche à base d'élastomère (6) comprend un élastomère silicone.

9. Membrane selon l'une quelconque des revendications 1 à 8, **caractérisée en ce que** la deuxième couche adhésive (5) comprend un gel silicone.

10. Membrane selon l'une quelconque des revendications 1 à 9, **caractérisée en ce que** son épaisseur est sensiblement comprise entre 0,25 mm et 2 mm.

11. Membrane selon l'une quelconque des revendications 1 à 10, **caractérisée en ce qu'**elle comprend des moyens permettant une certaine perméabilité à l'eau, à la sueur et à la vapeur à ladite membrane.

12. Membrane selon la revendication 11, **caractérisée en ce qu'**elle comporte des micro perforations d'un diamètre de quelques microns.

13. Utilisation de la membrane selon l'une quelconque des revendications 1 à 12, pour la fabrication de patchs esthétiques.

14. Utilisation de la membrane selon l'une quelconque des revendications 1 à 12, comme surface de prothèse, notamment faciale.
